# EUROPÄISCHE PATENTSCHRIFT

(11) **EP 3 013 315 B1**
(45) Veröffentlichungstag und Bekanntmachung des Hinweises auf die Patenterteilung: **05.10.2022**
(21) Anmeldenummer: 14732237.4
(22) Anmeldetag: 25.06.2014
(51) Int. Cl.: A61K 9/00, A61F 13/02, A61M 37/00, A61K 9/70

(54) **VORRICHTUNG MIT TRANSDERMALEM THERAPEUTISCHEM SYSTEM, POSITIONIER- UND PENETRATIONSHILFE**
DEVICE HAVING TRANSDERMAL THERAPEUTIC SYSTEM, POSITIONING AND PENETRATION AID
DISPOSITIF DOTÉ D'UN SYSTÈME THÉRAPEUTIQUE TRANSDERMIQUE, D'UNE AIDE AU POSITIONNEMENT ET D'UNE AIDE À LA PÉNÉTRATION

(30) Priorität: 25.06.2013 EP 13173554
(43) Veröffentlichungstag der Anmeldung: 04.05.2016
(73) Patentinhaber: LTS LOHMANN Therapie-Systeme AG, 56626 Andernach (DE)
(72) Erfinder: WORTMANN, Uwe, 35037 Marburg (DE); HADASCHIK, Roman, 99817 Eisenach (DE); HORSTMANN, Michael, 56564 Neuwied (DE)
(86) Internationale Anmeldenummer: PCT/EP2014/063373
(87) Internationale Veröffentlichungsnummer: WO 2014/207033

(56) Entgegenhaltungen:
- WO-A1-2012/075375
- WO-A2-02/19985
- US-A- 3 964 482

## Beschreibung

Die Erfindung betrifft eine Vorrichtung mit einem auf die Haut aufklebbaren Positionierrahmen und mit einem in eine den Positionierrahmen durchdringende Ausnehmung einsetzbaren transdermalem therapeutischem System, wobei die wirkstoffabgebenede Ausgabeseite des eingesetzten transdermalen therapeutischen Systems der Haut zugewandt ist.

Aus der WO 2006 / 131 931 A2 ist eine derartige Vorrichtung bekannt. Die Wirkstoffapplikation kann durch die Barriereeigenschaften der Haut behindert oder verhindert werden.

Nach den Druckschriften WO 2012/075375 A1, US 3,964,482, WO 02/19985 A2 erfolgt der Transport der therapeutischen Mittel immer durch Hohlnadeln hindurch. Bei der Anwendung müssen die Nadeln in der Haut des Patienten verbleiben. Alle diese Druckschriften erfordern damit den gleichzeitigen Einsatz der Hohlnadeln und der therapeutischen Mittel.

Der vorliegenden Erfindung liegt die Problemstellung zugrunde, die Wirkstoffübertragung des transdermalen therapeutischen Systems zu verbessern.

Diese Problemstellung wird mit den Merkmalen des Hauptanspruchs gelöst. Dazu umfasst die Vorrichtung eine in die Ausnehmung des Positionierrahmens einsetzbare und in dieser zentrierbare Werkzeugeinheit zur Erzeugung von Durchbrüchen zumindest in der obersten Schicht der Haut, sodass das transdermale therapeutische System nach dem Herausziehen der Werkzeugeinheit aus der Ausnehmung in die Ausnehmung eintauchbar ist. Die Werkzeugeinheit ermöglicht somit die Perforation des Stratum Corneum bzw. wird zur Erzeugung von Diffusionskanälen in der oberen Hautschicht eingesetzt.

Weitere Einzelheiten der Erfindung ergeben sich aus den Unteransprüchen und den nachfolgenden Beschreibungen schematisch dargestellter Ausführungsbeispiele.
- Figur 1:: Vorrichtung mit Positionier- und Penetrationshilfe sowie transdermalem therapeutischem System;
- Figur 2:: Vorrichtung nach Figur 1, auf die Haut aufgeklebt;
- Figur 3:: Eintauchen der Werkzeugeinheit;
- Figur 4:: Vorrichtung mit in die Haut eingedrungenen Werkzeugen;
- Figur 5:: Vorrichtung nach Figur 1 beim Einsatz der Penetrationshilfe;
- Figur 6:: Eintauchen des transdermalen therapeutischen Systems;
- Figur 7:: Vorrichtung nach Figur 1 beim Einsatz des transdermalen therapeutischen Systems;
- Figur 8:: Vorrichtung nach Figur 4 nach dem Abreißen des Positionierrahmens;
- Figur 9:: Verschließbarer Positionierrahmen;
- Figur 10:: Stempel mit Penetrationshilfe und transdermalem therapeutischem System;
- Figur 11:: Verschlossener Positionierrahmen;
- Figur 12:: Positionierrahmen mit Einführkegel.

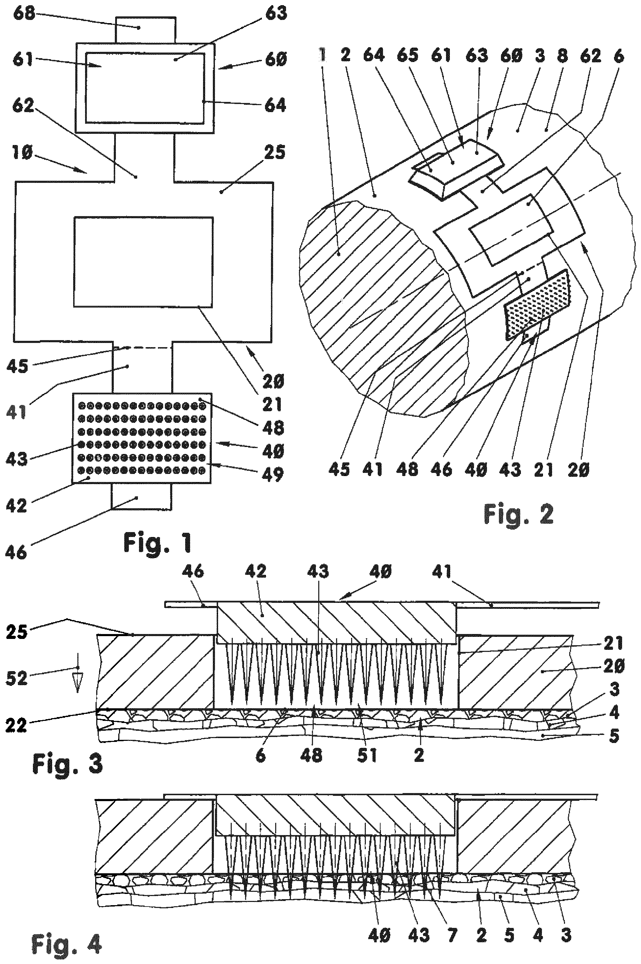

Die Figuren 1 - 7 zeigen eine Vorrichtung (10) mit einem Positionierrahmen (20), mit einer ein transdermales therapeutisches System (61) umfassenden Wirkstoffeinheit (60) und mit einer Werkzeugeinheit (40). Die Figur 1 zeigt hierbei die Vorrichtung vor dem Einsatz, wobei die Lager- und Transportschutzvorrichtungen bereits entfernt sind. In dieser Draufsicht liegt die Wirkstoffeinheit (60) oberhalb und die Werkzeugeinheit (40) unterhalb des Positionierrahmens (20). Sowohl die Wirkstoffeinheit (60) als auch die Werkzeugeinheit (40) sind in diesem Ausführungsbeispiel mittels jeweils einer z.B. als Filmgelenk ausgebildeten Verbindungslasche (41, 62) mit dem Positionierrahmen (20) verbunden.

Der Positionierrahmen (20) ist in der Draufsicht der Figur 1 ein rechteckiger Rahmen konstanter Dicke. Die Dicke ist beispielsweise geringer als drei Millimeter. Der Positionierrahmen (20) ist aus einem elastisch verformbaren Werkstoff hergestellt. Dies kann ein Kunststoff, z.B. ein Polymerwerkstoff sein. Er kann aus einer oder mehreren Komponenten hergestellt sein. Bei einem Mehrkomponentenwerkstoff, beispielsweise einem Zwei-Komponenten-Werkstoff, kann der in der Darstellung der Figur 1 dem Betrachter abgewandte Bereich des Positionierrahmens (20) einen geringeren Elastizitätsmodul aufweisen als der dem Betrachter zugewandte Bereich des Positionierrahmens (20). Als Werkstoffe für den Positionierahmen können beispiesweise Polyethylen, Silikon, TPE, Pharmagummi, Moosgummi oder Pappe, etc. eingesetzt werden.

Der Positionierrahmen (20) hat im Ausführungsbeispiel eine ihn durchdringende Ausnehmung (21) mit einer rechteckigen Querschnittsfläche. Diese Ausnehmung (21) ist beispielsweise symmetrisch zur vertikalen Mittenlängsebene der Vorrichtung (10) und symmetrisch zur vertikalen Mittenquerebene der Vorrichtung (10) angeordnet. Auf der dem Betrachter der Figur 1 abgewandten Unterseite (22) des Positionierrahmens (20) ist eine Klebeschicht angeordnet, vgl. Figur 3.

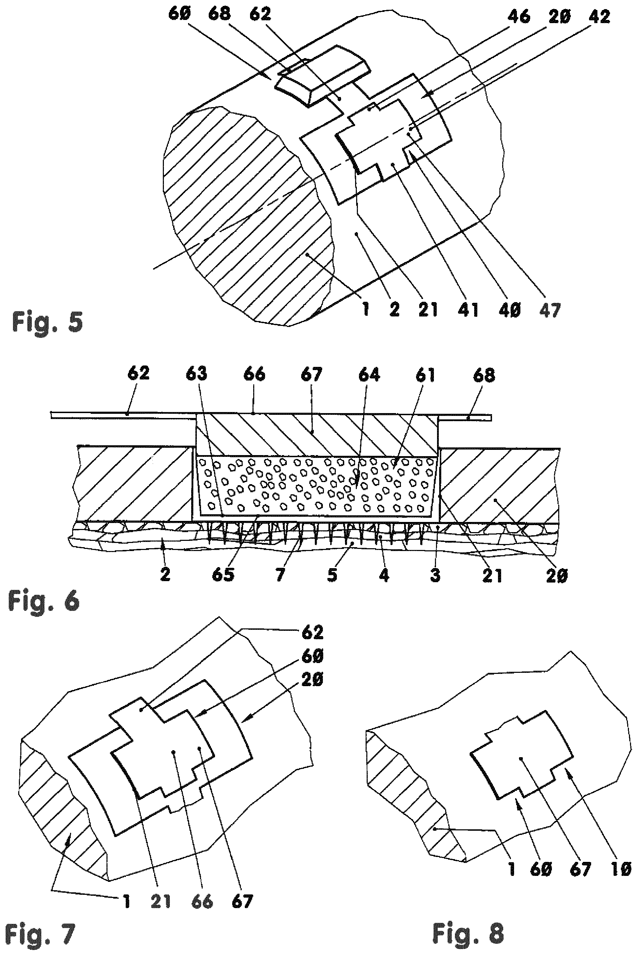

Die Wirkstoffeinheit (60) hat in der Draufsicht der Figur 1 ebenfalls eine rechteckige Querschnittsfläche. Diese Querschnittsfläche ist geringfügig kleiner als die Querschnittsfläche der Aussparung (21). Beispielsweise sind die Länge und die Breite der Querschnittsfläche der Wirkstoffeinheit (60) jeweils um einen zehntel Millimeter kleiner als die entsprechenden Abmessungen der Querschnittsfläche der Aussparung (21). Diese Differenz kann zwischen einem hundertstel Millimeter und einem Millimeter liegen. Die dem Betrachter der Figur 1 zugewandte Fläche der Wirkstoffeinheit (60) umfasst das transdermale therapeutische System (61) mit der wirkstoffabgebenden Ausgabeseite (63) des Wirkstoffreservoirs (64). An der hier oben liegenden Fläche der wirkstoffabgebensen Ausgabeseite (63) ist eine Klebeschicht (65) aufgebracht, vgl. Figuren 2 und 6. Die Rückseite (66) der Wirkstoffeinheit (60) bildet eine Trägerplatte (67).

Die Verbindungslasche (62) zwischen der Wirkstoffeinheit (60) und dem Positionierrahmen (20) ist aus einem biegbaren Werkstoff hergestellt. In der Darstellung der Figur 1 hat sie eine konstante Breite. Die Verbindungslasche (62) kann jedoch auch eine Einschnürung, eine Perforation, einen Einschnitt, etc. aufweisen. Diese Sollbruchstelle ist beispielsweise in einem Abstand von fünf Millimetern vom transdremalen therapeutischen System (61) angeordnet.

Die Werkzeugeinheit (40) hat in der in der Figur 1 dargestellten Draufsicht eine rechteckige Querschnittsfläche. Diese Querschnittsfläche ist geringfügig kleiner als die Querschnittsfläche der Ausnehmung (21). Beispielsweise entspricht die Querschnittsfläche der Werkzeugeinheit (40) der Querschnittsfläche der Wirkstoffeinheit (60). Die Werkzeugeinheit (40) umfasst in der Darstellung der Figur 1 einen Werkzeugträger (42) und eine Vielzahl von Werkzeugen (43). Der Werkzeugträger (42) ist plattenartig aufgebaut. Sein Werkstoff hat beispielsweise einen höheren Elastizitätsmodul als der Werkstoff des dem Betrachter abgewandten Bereichs des Positionierrahmens (20).

Die Werkzeuge (43) sind in den Darstellungen der Figuren 1 - 4 aus dem Werkzeugträger (42) herausragende Nadeln (43). Im Ausführungsbeispiel umfasst die Werkzeugeinheit (40) 90 Nadeln (43). Die Anzahl der Nadeln (43) kann beispielsweise zwischen zehn und zweitausend betragen. Im Ausführungsbeispiel ist die Vielzahl von Nadeln (43) in einem regelmäßgen Gitter, einem Array, angeordnet. Auch eine kreisförmige, ovale, vieleckige etc. Anordnung ist denkbar.

Die einzelnen Nadeln (43) stehen um den gleichen Betrag aus dem Werkzeugträger (42) heraus, so dass ihre Spitzen zumindest annähernd in einer Ebene liegen. Die Länge, mit der die einzelnen Nadeln (43) aus dem Werkzeugträger (42) herausstehen, ist im Ausführungsbeispiel kürzer als die Dicke des Positionierrahmens (20). Beispielsweise beträgt diese Länge 2,5 Millimeter. Die damit mögliche Eindringtiefe der Nadeln (43) in der Haut (2) liegt beispielsweise bei 0,4 bis 0,8 mm. Andere Eindringtiefen können durch allgemein bekannte Veränderungen an der Vorrichtung eingestellt werden. Die aus Vollmaterial ausgebildeten Nadeln (43) sind z.B. aus einem austenitischen Stahl hergestellt. Der Arbeitsraum (51) der Werkzeugeinheit (40) ist damit an der Stirnseite (48) der Werkzeugeinheit (40) angeordnet. Die Querschnittsfläche des Arbeitsraums (51) wird hierbei durch die die Werkzeuge (43) umgebende Hüllfläche begrenzt. Diese Querschnittsfläche ist kleiner als die hierzu parallele Querschnittsfläche der Ausnehmung (21)

Die Werkzeugeinheit (40) kann auch eine Laservorrichtung, Ultraschallvorrichtung oder Elektroden aufweisen. Diese Vorrichtungen sind alternativ oder zusätzlich zu den Nadeln (43) denkbar.

Die Verbindungslasche (41), die die Werkzeugeinheit (40) mit dem Positionierrahmen (20) verbindet, ist ähnlich ausgebildet wie die Verbindungslasche (62) zwischen der Wirkstoffeinheit (60) und dem Positionierrahmen (20). Sie weist eine Perforation (45) auf, die beispielsweise in einem Abstand von 5 Millimetern vom Positionierrahmen (20) angeordnet ist. Anstatt der oder zusätzlich zur Perforation (45) ist auch eine Einschnürung, ein Einschnitt, etc. denkbar.

Sowohl die Wirkstoffeinheit (60) als auch die Werkzeugeinheit (40) weisen im Ausführungsbeispiel eine nach außen weisende Grifflasche (46, 68) auf. Gegebenenfalls kann auch der Positionierrahmen (20) eine oder mehrere Grifflaschen aufweisen.

In der Darstellung der Figur 2 ist die Vorrichtung (10) auf die Haut (2) des Patienten, z.B. auf einen Arm (1), aufgebracht. Hierfür ist der Positionierrahmen (20) nach dem Abziehen einer Schutzfolie auf die Haut (2) aufgeklebt. Der Positionierrahmen (20) ist der Gestalt des Arms (1) angepasst, so dass er vollflächig auf dem Arm (1) aufgeklebt ist. Der Positionierrahmen (20) umgibt einen Hautausschnitt (6). Gegebenenfalls ist dieser Hautausschnitt (6) aufgrund des Verklebens mit dem bereichsweise verformungssteifen Positionierrahmen (20) gespannt.

Die Wirkstoffeinheit (60) hängt an der Verbindungslasche (62). Die wirkstoffabgebende Ausgabeseite (63) des Wirkstoffreservoirs (64) zeigt in die der Haut (2) abgewandte Seite, im Ausführungsbeispiel also nach oben. In der Darstellung der Figur 2 liegt die Wirkstoffeinheit (60) mit ihrer Rückseite (66) auf dem Arm (1) auf. Gegebenenfalls kann beim Aufkleben des Positionierrahmens (20) sowohl die Abgabeseite (63) des Wirkstoffreservoirs (64) als auch die Werkzeugeinheit (40) mittels abnehmbarer Schutzvorrichtungen geschützt sein.

Die Werkzeugeinheit (40) hängt an der Verbindungslasche (41), wobei die Werkzeuge (43) in die der Haut (2) abgewandte Richtung zeigen. Beispielsweise liegt der Werkzeugträger (42) mit seiner Rückseite (47) auf der Haut (2) auf und schmiegt sich an dieser zumindest bereichsweise an.

Nach dem Entfernen der Schutzfolie wird die Werkzeugeinheit (40) um das Filmgelenk der Verbindungslasche (41) geschwenkt. Hierbei wird die Werkzeugeinheit (40) mittels der Grifflasche (46) gehalten und geführt. Die Werkzeuge (43) werden in der Eintauchrichtung (52) in die Ausnehmung (21) eingetaucht, vgl. Figur 3. Die Verbindungslasche (41) verbindet weiterhin die Werkzeugeinheit (40) mit dem Positionierrahmen (20). Gegebenenfalls kann der Positionierrahmen (20) und/oder der Werkzeugträger (42) Einführschrägen oder andere Zentrierelemente aufweisen. Diese Zentrierelemente können z.B. Nasen, Stifte, Löcher, Ausnehmungen, etc. sein, die sowohl am Positionierrahmen (20) als auch an der Werkzeugeinheit (40) angeordnet sein können. Beim weiteren Einführen wird der Werkzeugträger (42) mittels der Ausnehmung (21) zentriert. Beim Beginn der Zentrierung sind die Werkzeuge (43) beabstandet von der Haut (2), vgl. Figur 3. Der an der Stirnseite (48) der Werkzeugeinheit (40) angeordnete Arbeitsraum (51) ist noch ohne Werkzeugeingriff. Die auf die Haut (2) projizierte Fläche des Arbeitsraums (51) ist kleiner als die vom Positionierrahmen (20) umgebene Querschnittsfläche der Ausnehmung (21).

Die Werkzeugeinheit (40) wird in die Haut (2) eingepresst, vgl. Figur 4. Hierbei durchdringen die Werkzeuge (43) die obersten Hautschichten (3, 4, 5) und bilden Durchbrüche (7) in diesen Hautschichten (3, 4, 5). In der Darstellung der Figur 4 sind die obersten beiden Hautschichten (3, 4) vollständig durchdrungen. Es ist aber auch denkbar, dass die Nadeln (43) nur die oberste Hautschicht (3), die Epidermis (3), durchdringen. Beim Einpressen der Werkzeuge (43) in die Haut (2) wird die Werkzeugeinheit (40) weiterhin mittels des Positionierrahmens (20) zentriert.

Die Figur 5 zeigt die Vorrichtung (10) mit in die Haut (2) eingepresster Werkzeugeinheit (40). Die Rückseite (47) des Werkzeugträgers (42) zeigt nach außen, wobei der Werkzeugträger (42) nach oben über den Positionierrahmen (20) herausragt. Die Werkzeugeinheit (40) sitzt innerhalb der Ausnehmung (21) des Positionierrahmens (20). Gegebenenfalls kann das Eindrücken der Werkzeugeinheit (40) mittels eines Anschlags begrenzt sein. Beispielsweise können die Verbindungslasche (41) und/oder die Grifflasche (46) einen derartigen Anschlag bilden oder umfassen. Die Werkzeugeinheit (40) kann koaxial zur Krümmung der Haut (2) und/oder des Positionierrahmens (20) verformt sein. Die Werkzeugeinheit (40) und der Positionierrahmen (20) sind mittels der Verbindungslasche (41) verbunden. Die Grifflasche (46) der Werkzeugeinheit (40) zeigt in Richtung der Wirkstoffeinheit (60) mit dem transdermalen therapeutischen System (61). Die Wirkstoffeinheit (60) liegt gegenüber der Darstellung der Figur 2 unverändert auf dem Arm (1).

Zum Herausziehen der Werkzeugeinheit (40) aus der Ausnehmung (21) greift der Anwender z.B. mit einer Hand die Grifflasche (46) und zieht die Werkzeugeinheit (40) entgegen der Eintauchrichtung (52). Hiernach kann die Werkzeugeinheit (40) entlang der Perforation (45) abgerissen und entsorgt werden.

Nach dem Abnehmen der Schutzfolie wird die Wirkstoffeinheit (60) mit dem transdermalen therapeutischen System (61) in die Ausnehmung (21) eingeschwenkt. Hierbei greift der Anwender z.B. die Grifflasche (68) und setzt das transdermale therapeutische System (61) so in die Ausnehmung (21), dass die Trägerplatte (67) in der Ausnehmung (21) zentriert ist. Das transdermale therapeutische System (61) berührt in diesem Zustand noch nicht die Haut (2) des Patienten, vgl. Figur 6. Gegebenenfalls kann die Trägerplatte (67) Zentrierschrägen aufweisen. Auch die in Zusammenhang mit der Werkzeugeinheit (40) genannten Zentrierelemente sind denkbar. Die Dicke des Wirkstoffreservoirs (64) des transdermalen therapeutischen Systems (61) ist geringer als die Dicke des Positionierrahmens (20).

Beim weiteren Eindrücken des transdermalen therapeutischen Systems (61) wird die wirkstoffabgebende Ausgabeseite (63) auf den freiliegenden Bereich (6) der Haut (2) aufgepresst. Gleichzeitig verklebt das transdermale therapeutische System (61) mit der Haut (2). Beim weiteren Eindrücken wird die Wirkstoffeinheit (60) weiter mittels der Ausnehmung (21) zentriert. Gegebenenfalls ist der Hub des Eindrückens mittels eines Anschlags begrenzt. Die Verbindungslasche (62) und/oder die Grifflasche (68) können einen derartigen Anschlag bilden oder umfassen.

In der Figur 7 ist die Vorrichtung (10) mit dem in die Ausnehmung (21) eingeführten und auf die Haut (2) aufgepressten transdermalen therapeutischen System (61) dargestellt. Die Trägerplatte (67) der Wirkstoffeinheit (60) steht geringfügig über den Positionierrahmen (20) über. Der Wirkstoff wird aus dem Wirkstoffbehälter (64) durch die Ausgabeseite (63) hindurch in die Haut (2) abgegeben. Hierbei dringt der Wirkstoff durch die mittels der Penetrationshilfe (40) erzeugten Durchbrüche (7) hindurch in die Hautschichten (4, 5) unterhalb der Hornhaut (3). Damit erfolgt eine wirksame subkutane Wirkstoffeinbringung.

Nach dem Aufsetzen und Verkleben des transdermalen therapeutischen Systems (61) kann der Positionierrahmen (20) entfernt werden. Hierzu kann er beispielsweise an einer Ecke oder an einem Griffstück gegriffen werden und von der Haut (2) abgezogen werden. Das transdermale therapeutische System (61) verbleibt hierbei auf der Haut (2). Beim Abziehen des Positionierrahmens (20) wird die Verbindungslasche (62) beispielsweise an einer Perforation abgerissen. Der Positionierrahmen (20) kann nun entsorgt werden. In der Figur 8 ist der Arm (1) des Patienten mit der auf die Wirkstoffeinheit (60) reduzierten Vorrichtung (10) dargestellt. Mittels des mithilfe des Positionierrahmens (20) positionierten transdermalen therapeutischen Systems (61) wird weiter Wirkstoff in die Haut (2) des Patienten eingebracht. Die Position des transdermalen therapeutischen Systems (61) kann zusätzlich mittels eines Pflasters gesichert werden. Gegebenenfalls kann beim Einsatz eines derartigen Pflasters auf die Klebeschicht (65) auf der wirkstoffabgebenden Ausgabeseite (63) verzichtet werden.

Bei einer Perforation der Haut (2) mittels Laserstrahlen oder Ultraschall erfolgt ebenfalls eine Zentrierung der Werkzeugeinheit (40) im Positionierrahmen (21). Das Zentrieren der Wirkstoffeinheit (60) und das Einbringen des Wirkstoffs erfolgen, wie oben beschrieben.

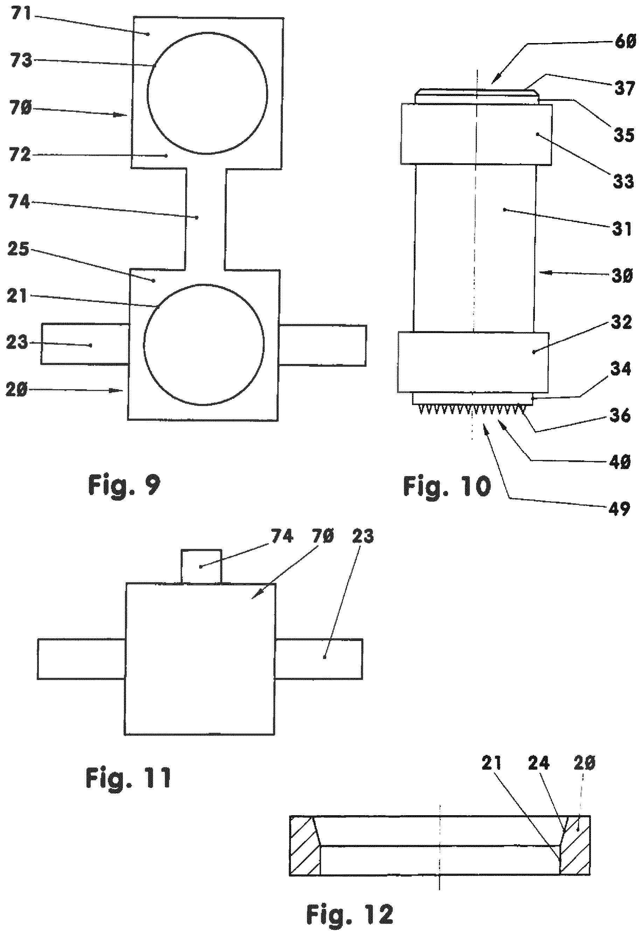

Die Figuren 9 - 11 zeigen eine weitere Ausführungsform der Vorrichtung (10). In der Figur 9 ist ein Positionierrahmen (20) mit einem Deckel (70) dargestellt. Dieser Positionierrahmen (20) mit z.B. quadratischer Grundfläche hat eine Ausnehmung (21) mit einer kreisförmigen Querschnittsfläche. Seitlich am Positionierrahmen (20) sind zwei Pflaster (23) angeordnet, mit denen der Positionierrahmen (20) auf die Haut (2) des Patienten aufklebbar ist. Der Werkstoff und die Materialdicke des Positionierrahmens (20) entsprechen den im Zusammenhang mit dem ersten Ausführungsbeispiel angegebenen Daten.

Der Deckel (70) hat in diesem Ausführungsbeispiel eine quadratische Grundfläche. Die in der Figur 9 dem Betrachter zugewandte Vorderseite (71) der Trägerplatte (72) des Deckels (70) trägt einen zentral angeordneten zylinder- oder kegelstumpfförmigen Zentrieransatz (73). Die maximale Querschnittsfläche des Zentrieransatzes (73) ist kleiner als die Querschnittsfläche der Ausnehmung (21). Die Maßdifferenz zwischen dem Zentrieransatz (73) und der Ausnehmung (21) kann den im Zusammenhang mit dem ersten Ausführungsbeispiel genannten Maßdifferenzen entsprechen.

Der Deckel (70) ist mittels einer biegbaren Verbindungslasche (74) mit dem Positionierrahmen (20) verbunden. Diese Verbindungslasche (74) ist so aufgebaut wie die im Zusammenhang mit dem ersten Ausführungsbeispiel beschriebenen Verbindungslaschen (41, 62).

Die Figur 10 zeigt als Teil der Vorrichtung (10) einen Stempel (30). Dieser hat beispielsweise einen zylinderförmigen Grundkörper (31). An beiden Enden ist jeweils ein den Grundzylinder (31) umgebender Verstärkungsring (32, 33) angeordnet. Der jeweilige Verstärkungsring (32, 33) ist um einen Eintauchbereich (34, 35) beabstandet zur jeweiligen Stirnseite (36, 37) des Grundzylinders (31). Die in der Darstellung der Figur 10 untenliegende Stirnseite (36) trägt eine Werkzeugeinheit (40). Diese umfasst eine Vielzahl von Nadeln (43). Die Anzahl und die Ausbildung der Nadeln (43) entsprichen der Anzahl und der Ausbildung der im Zusammenhang mit dem ersten Ausführungsbeispiel beschriebenen Nadeln (43). Anstatt eines Nadelarrays (49) kann die Werkzeugeinheit (40) eine Laserbaugruppe, eine Ultraschallbaugruppe, Elektroden, etc. umfassen. Die Werkzeugeinheit (40) kann dann an oder im Stempel (30) angeordnet sein. Im Ausgangszustand ist die Werkzeugeinheit (40) beispielsweise mittels einer Schutzfolie geschützt.

Die der Werkzeugeinheit (40) entgegengesetzt angeordnete Stirnseite (37) des Stempels (30) trägt eine Wirkstoffeinheit (60) mit einem transdermalen therapeutischen System (61). In diesem Ausführungsbeispiel kann die wirkstoffabgebende Ausgabeseite (63) des transdermalen therapeutischen Systems (61) sowohl mit als auch ohne eine Kleberschicht ausgeführt sein.

Nach dem Aufkleben des Positionierrahmens (20) auf die Haut (2) wird der Stempel (30) mit der Werkzeugeinheit (40) voraus in die Ausnehmung (21) des Positionierrahmens (20) eingeführt. Hierbei wird der Eintauchbereich (34) in der Ausnehmung (21) des Positionierrahmens (20) zentriert. Die Zentrierung kann so ausgebildet sein, wie im Zusammenhang mit dem ersten Ausführungsbeispiel beschrieben. Beim weiteren Eindrücken durchdringen auch in diesem Ausführungsbeispiel die Werkzeuge (43) der Werkzeugeinheit (40) zumindest die oberste Schicht (3) der Haut (2). Die Haut (2) wird perforiert. Die Werkzeugeinheit (40) kann beispielsweise so weit auf die Haut (2) aufgepresst werden, bis der Verstärkungsring (32) auf dem Positionierrahmen (20) aufliegt. Der Verstärkungsring (32) bildet hier zusammen mit dem Positionerrahmen (20) einen Hubanschlag für die Werkzeugeinheit (40).

Der Stempel (30) wird nun wieder herausgezogen. Gegebenenfalls kann die Werkzeugeinheit (40) mit einer Schutzkappe versehen werden. Als Nächstes wird der Stempel (40) mit der anderen Stirnseite (37), auf der das transdermale therapeutische System (61) angeordnet ist, in die Ausnehmung (21) des Positionierrahmens (20) eingeführt. Hierbei wird der Eintauchbereich (35) in der Ausnehmung (21) des Positionierrahmens (20) eingesetzt und zentriert. Beim weiteren Einschieben berührt das transdermale therapeutische System (61) mit seiner wirkstoffabgebenden Ausgabeseite (63) den freiliegenden Bereich (6) der Haut (2). Das weitere Einpressen des Stempels (30) fördert das Ausbringen des Wirkstoffs aus dem Wirkstoffbehälter (64) durch die Hautdurchbrüche (7) hindurch in die Hautschichten (4, 5) unterhalb der Hornhaut (3). Das Einpressen erfolgt so lange, bis der Verstärkungsring (33) am Positionierrahmen (20) anliegt und gegebenenfalls dort während eines vorgegebenen Zeitintervalls verharrt. Der Hubanschlag verhindert das weitere Einschieben des transdermalen therapeutischen Systems (61) und damit eine unkontrollierte Wirkstoffabgabe.

Nach dem Entnehmen des Stempels (30) kann der Positionierrahmen mittels des Deckels (70) verschlossen werden, vgl. Figur 11. Hierbei wird der Deckel mittels des Zentrieransatzes (73) in der Ausnehmung (21) zentriert. Gegebenenfalls kann der Deckel mit der Haut (2) verkleben. Je nach Aufbau des Positionierrahmens (20) kann dieser jetzt von der Haut (2) abgezogen werden. Der Klebstoff zum Verkleben des Positionierrahmens (20) mit der Haut (2) kann eine andere Formulierung aufweisen als der Klebstoff zum Verkleben des transdermalen therapeutischen Systems (61) mit der Haut (2). Beispielsweise erfordert der Klebstoff des Positionierrahmens (20) einen geringeren Kraftaufwand zum Lösen des Positionierrahmens (20) von der Haut (2) als der Klebstoff des transdermalen therapeutischen Systems (61).

Der Stempel (30) kann auch derart ausgebildet sein, dass das transdermale therapeutische System (61) mittels des Stempels (30) in der Ausnehmung (21) abgelegt wird. Auch der Einsatz eines anderen Werkzeugs zum Einlegen des transdermalen therapeutischen Systems (61) in der Ausnehmung (21) ist denkbar. In allen Fällen weist nach dem Einsetzen die wirkstoffabgebende Ausgabeseite (63) des trandermalen therapeutischen Systems (61) in Richtung der Haut (2).

Nach dem Einlegen des transdermalen therapeutischen Systems (61) wird der Deckel (70) an der Ausnehmung (21) zentriert und verschlossen. Das Ausbringen des Wirkstoffs aus dem Wirkstoffbehälter (64) erfolgt auch in diesem Fall durch die in die Haut (2) eingebrachten Durchbrüche (7) hindurch.

Das transdermale therapeutische System (61) kann auch in den Deckel (70) integriert sein. Nach der Perforation der Haut (2) mittels der Werkzeugeinheit (40) wird der Deckel (70) in die Ausnehmung (21) geschwenkt, wie im Zusammenhang mit dem ersten Ausführungsbeispiel beschrieben.

In der Figur 12 ist ein Positionierrahmen (20) dargestellt, dessen Ausnehmung (21) einen Einführkegel (24) aufweist. Die gedachte Kegelspitze liegt unterhalb der Hautoberfläche (8), vgl. Figur 2. Der Spitzenwinkel des Einführkegels (24) beträgt beispielsweise 30 Grad. Dieser Winkel kann zwischen 5 Grad und 45 Grad betragen. Die Länge dieser Anfasung ist kürzer oder gleich der Hälfte der Länge der Ausnehmung (21) normal zur Haut (2). Bei einer rechteckigen Querschnittsfläche der Ausnehmung (21) sind statt eines Einführkegels (24) Einführschrägen ausgebildet. Die Einführ- und Zentrierelemente können auch auf der der Haut (2) abgewandten Oberseite (25) des Positionierrahmens (20) angeordnet sein.

Selbstverständlich ist es auch denkbar, die verschiedenen genannten Ausführungsformen miteinander zu kombinieren.

### Bezugszeichenliste:

- 1: Arm
- 2: Haut
- 3: Epidermis, Hornhaut, oberste Hautschicht
- 4: zweite Hautschicht
- 5: dritte Hautschicht
- 6: Hautausschnitt, freiliegender Bereich
- 7: Durchbrüche
- 8: Hautoberfläche

- 10: Vorrichtung

- 20: Positionierrahmen
- 21: Ausnehmung
- 22: Unterseite von (20)
- 23: Pflaster
- 24: Einführkegel, Zentrierelement
- 25: Oberseite von (20)

- 30: Stempel
- 31: Grundkörper, Grundzylinder
- 32: Verstärkungsring, werkzeugseitig
- 33: Verstärkungsring, wirkstoffseitig
- 34: Eintauchbereich, werkzeugseitig
- 35: Eintauchbereich, wirkstoffseitig
- 36: Stirnseite, werkzeugseitig
- 37: Stirnseite, wirkstoffseitig

- 40: Werkzeugeinheit, Penetrationshilfe
- 41: Verbindungslasche
- 42: Werkzeugträger
- 43: Werkzeuge, Nadeln
- 45: Perforation
- 46: Grifflasche
- 47: Rückseite
- 48: Stirnseite
- 49: Nadelarray

- 51: Arbeitsraum
- 52: Eintauchrichtung

- 60: Wirkstoffeinheit
- 61: transdermales therapeutisches System
- 62: Verbindungslasche
- 63: Abgabeseite, wirkstoffabgebende Ausgabeseite
- 64: Wirkstoffreservoir, Wirkstoffbehälter
- 65: Klebeschicht
- 66: Rückseite
- 67: Trägerplatte
- 68: Grifflasche

- 70: Deckel
- 71: Vorderseite
- 72: Trägerplatte
- 73: Zentrieransatz
- 74: Verbindungslasche

## Patentansprüche

1. Vorrichtung (10) mit einem auf die Haut (2) aufklebbaren Positionierrahmen (20) und mit einem in eine den Positionierrahmen (20) durchdringende Ausnehmung (21) einsetzbaren transdermalem therapeutischem System (61),
wobei die wirkstoffabgebenede Ausgabeseite (63) des eingesetzten transdermalen therapeutischen Systems (61) der Haut (2) zugewandt ist,
**dadurch gekennzeichnet,**
**dass** die Vorrichtung (10) eine in die Ausnehmung (21) des Positionierrahmens (20) einsetzbare und in dieser zentrierbare Werkzeugeinheit (40) zur Erzeugung von Durchbrüchen (7) zumindest in der obersten Schicht (3) der Haut (2) umfasst,
sodass das transdermale therapeutische System (61) nach dem Herausziehen der Werkzeugeinheit (40) aus der Ausnehmung (21) in die Ausnehmung (21) eintauchbar ist.

2. Vorrichtung (10) nach Anspruch 1,
**dadurch gekennzeichnet,**
**dass** die wirkstoffabgebende Ausgabeseite (63) des transdermalen therapeutischen Systems (61) eine Klebeschicht (65) zur Verklebung mit der Haut (2) trägt.

3. Vorrichtung nach Anspruch 1 oder 2,
**dadurch gekennzeichnet,**
**dass** die Dicke des den Wirkstoff des transdermalen therapeutischen Systems (61) enthaltenen Wirkstoffbehälters (64) kleiner ist als die Dicke des Positionierrahmens (21).

4. Vorrichtung (10) nach einem der vorhergehenden Ansprüche,
**dadurch gekennzeichnet,**
**dass** zumindest eine das transdermale therapeutische System (61) umfassende Wirkstoffeinheit (60) mittels einer biegbaren Verbindungslasche (62) mit dem Positionierrahmen (20) verbunden ist.

5. Vorrichtung (10) nach Anspruch 4,
**dadurch gekennzeichnet,**
**dass** die Wirkstoffeinheit (60) eine dem transdermalen therapeutischen System (61) abgewandte Trägerplatte (67) aufweist.

6. Vorrichtung (10) nach einem der vorhergehenden Ansprüche,
**dadurch gekennzeichnet,**
**dass** die Ausnehmung (21) einen Einführkegel (24) oder Einführschrägen aufweist.

7. Vorrichtung (10) nach einem der vorhergehenden Ansprüche,
**dadurch gekennzeichnet,**
**dass** die Werkzeuge (43) der Werkzeugeinheit (40) als Nadeln (43) ausgebildet sind.

8. Vorrichtung nach Anspruch 7,
**dadurch gekennzeichnet,**
**dass** die Werkzeuge (43) in einem Werkzeugträger (42) gehalten sind.

9. Vorrichtung nach Anspruch 8,
**dadurch gekennzeichnet,**
**dass** die aus dem Werkzeugträger (42) herausstehende Länge der Werkzeuge (43) kürzer ist als die Dicke des Positionierrahmens (20).

10. Vorrichtung nach Anspruch 1,
**dadurch gekennzeichnet,**
**dass** die Werkzeugeinheit (40) an oder in einem Stempel (30) angeordnet ist.

## Claims

1. Device (10) with a positioning frame (20) which can be affixed to the skin (2) and with a transdermal therapeutic system (61) which can be inserted into a recess (21) passing through the positioning frame (20), the active substance delivery side (63) of the inserted transdermal therapeutic system (61) facing the skin (2), **characterized in that** the device (10) comprises a tool unit (40) which can be inserted into the recess (21) of the positioning frame (20) and centered in said recess in order to produce openings (7) at least in the uppermost layer (3) of the skin (2), such that the transdermal therapeutic system (61) is insertable into the recess (21) after the tool unit (40) has been withdrawn from the recess (21).

2. Device (10) according to Claim 1, **characterized in that** the active substance delivery side (63) of the transdermal therapeutic system (61) carries an adhesive layer (65) for adhering to the skin (2).

3. Device according to Claim 1 or 2, **characterized in that** the thickness of the active substance container (64) containing the active substance of the transdermal therapeutic system (61) is less than the thickness of the positioning frame (21).

4. Device (10) according to one of the preceding claims, **characterized in that** at least one active substance unit (60) comprising the transdermal therapeutic system (61) is connected to the positioning frame (20) by means of a bendable connecting tab (62).

5. Device (10) according to Claim 4, **characterized in that** the active substance unit (60) has a support plate (67) facing away from the transdermal therapeutic system (61).

6. Device (10) according to one of the preceding claims, **characterized in that** the recess (21) has an insertion taper (24) or insertion bevels.

7. Device (10) according to one of the preceding claims, **characterized in that** the tools (43) of the tool unit (40) are designed as needles (43).

8. Device according to Claim 7, **characterized in that** the tools (43) are held in a tool support (42).

9. Device according to Claim 8, **characterized in that** the length of the tools (43) protruding from the tool support (42) is shorter than the thickness of the positioning frame (20).

10. Device according to Claim 1, **characterized in that** the tool unit (40) is arranged on or in a stamp (30) .

## Revendications

1. Dispositif (10) comprenant un cadre de positionnement (20) pouvant être collé sur la peau (2) et un système thérapeutique transdermique (61) qui peut être inséré dans un évidement (21) traversant le cadre de positionnement (20), le côté (63) du système thérapeutique transdermique (61) utilisé qui libère le principe actif étant dirigé vers la peau (2), **caractérisé en ce que**
le dispositif (10) comprend une unité d'outils (40) qui peut être insérée dans l'évidement (21) du cadre de positionnement (20), qui peut être centrée dans celuici et qui est destinée à générer des passages (7) au moins dans la couche supérieure (3) de la peau (2) de sorte que le système thérapeutique transdermique (61) puisse être plongé dans l'évidement (21) après que l'unité d'outils (40) a été retirée de l'évidement (21) .

2. Dispositif (10) selon la revendication 1, **caractérisé en ce que**
le côté du système thérapeutique transdermique (61) qui libère le principe actif (63) porte une couche adhésive (65) permettant d'adhérer à la peau (2).

3. Dispositif selon la revendication 1 ou 2, **caractérisé en ce que**
l'épaisseur du récipient de principe actif (64) contenant le principe actif du système thérapeutique transdermique (61) est inférieure à l'épaisseur du cadre de positionnement (21).

4. Dispositif (10) selon l'une des revendications précédentes,
**caractérisé en ce que**
au moins une unité de principe actif (60) comprenant le système thérapeutique transdermique (61) est reliée au cadre de positionnement (20) au moyen d'une attache de liaison flexible (62).

5. Dispositif (10) selon la revendication 4,
**caractérisé en ce que**
l'unité de principe actif (60) comporte une plaque de support (67) dirigée à l'opposé du système thérapeutique transdermique (61).

6. Dispositif (10) selon l'une des revendications précédentes,
**caractérisé en ce que**
l'évidement (21) comporte un cône d'insertion (24) ou des biseaux d'insertion.

7. Dispositif (10) selon l'une des revendications précédentes,
**caractérisé en ce que**
les outils (43) de l'unité d'outils (40) sont conçus sous la forme d'aiguilles (43).

8. Dispositif selon la revendication 7,
**caractérisé en ce que**
les outils (43) sont maintenus dans un porte-outils (42) .

9. Dispositif selon la revendication 8,
**caractérisé en ce que**
la longueur des outils (43), au-delà du porte-outils (42), est inférieure à l'épaisseur du cadre de positionnement (20).

10. Dispositif selon la revendication 1,
**caractérisé en ce que**
l'unité d'outils (40) est disposée sur ou dans un piston (30).
